(19) Europäisches Patentamt
European Patent Office
Office européen des brevets

(11) **EP 3 417 869 B1**

(12) **EUROPEAN PATENT SPECIFICATION**

(45) Date of publication and mention
of the grant of the patent:
**12.02.2025 Bulletin 2025/07**

(21) Application number: **17753532.5**

(22) Date of filing: **17.02.2017**

(51) International Patent Classification (IPC):
*A61K 36/899* (2006.01)    *A23L 33/105* (2016.01)
*A61K 31/337* (2006.01)    *A61K 45/06* (2006.01)
*A61P 7/06* (2006.01)     *A61P 43/00* (2006.01)
*A23L 2/02* (2006.01)     *A23L 2/52* (2006.01)

(52) Cooperative Patent Classification (CPC):
**A23L 33/105; A23L 2/02; A23L 2/52; A61K 36/899;
A61P 7/06; A61P 43/00**

(86) International application number:
**PCT/KR2017/001813**

(87) International publication number:
**WO 2017/142371 (24.08.2017 Gazette 2017/34)**

(54) **COMPOSITION CONTAINING PHRAGMITIS RHIZOMA EXTRACT AS ACTIVE INGREDIENT FOR PREVENTING, AMELIORATING, OR TREATING DISEASE ATTRIBUTED TO SIDE EFFECT OF ANTICANCER AGENT**

ZUSAMMENSETZUNG MIT PHRAGMITIS-RHIZOMA-EXTRAKT ALS WIRKSTOFF ZUR VERHINDERUNG, LINDERUNG, ODER BEHANDLUNG VON KRANKHEITEN IM ZUSAMMENHANG MIT NEBENWIRKUNGEN EINES ANTIKREBSMITTELS

COMPOSITION CONTENANT UN EXTRAIT DE PHRAGMITIS RHIZOMA EN TANT QUE PRINCIPE ACTIF SERVANT À PRÉVENIR, AMÉLIORER OU TRAITER UNE MALADIE ATTRIBUÉE À UN EFFET SECONDAIRE D'AGENT ANTICANCÉREUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.02.2016 KR 20160019339**

(43) Date of publication of application:
**26.12.2018 Bulletin 2018/52**

(73) Proprietor: **Korea Institute of Oriental Medicine
Daejeon 34054 (KR)**

(72) Inventors:
• **KIM, Jinhee
  Daejeon 34069 (KR)**
• **CHO, Eun Sang
  Daejeon 35282 (KR)**
• **KIM, No Soo
  Daejeon 34053 (KR)**

• **BANG, Ok-Sun
  Daejeon 34033 (KR)**
• **LEE, You Jin
  Daejeon 34679 (KR)**
• **KIM, Young-Ah
  Daegu 41965 (KR)**
• **HUH, Eunna
  Gimpo-si
  Gyeonggi-do 10089 (KR)**

(74) Representative: **Kraus & Lederer PartGmbB
Thomas-Wimmer-Ring 15
80539 München (DE)**

(56) References cited:
WO-A2-2010/035262    WO-A2-2011/132986
WO-A2-2013/048140    CN-A- 103 239 636
KR-A- 20110 126 384    KR-A- 20110 126 384
KR-A- 20120 121 813    KR-A- 20140 015 199
KR-A- 20160 054 669

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

TECHNICAL FIELD

The present invention relates to a composition containing Phragmitis Rhizoma extract as an active ingredient for prevention, amelioration, or treatment of a disorder caused by side effect of certain anticancer agents.

BACKGROUND ART

Cancer is the first or second most common causes of death in South Korea. Cancer is response for the death of almost 30% of the people who died in their 50s or 60s in South Korea. As a cancer therapy, a surgical operation, radiotherapy, and chemotherapy are most widely employed. In particular, as chemotherapy, many efforts have been made to develop an anticancer agent that completely eradicates most types of cancer, although an anticancer agent working as a true therapeutic agent is yet to be developed. Most chemotherapeutic agents of current medicine just remains as an agent for extending survival for a short period of time.

The anticancer agent used for chemotherapy interrupts metabolic pathways of cancer cells by directly working on DNAs and blocking replication, transcription, or translational process of DNAs, or preventing synthesis of nucleic acid precursors to inhibit cell division, and thus exhibiting cytotoxicity. However, anticancer agents that are currently used have no selectivity for specific cancer, and thus they have a characteristic that the therapeutic effect on cancer cells and toxic effect on normal cells are exhibited simultaneously. Furthermore, the toxicity caused by administration of an anticancer agent includes various side effects such as cytopenia of white blood cells, platelets, red blood cells, or the like resulting from bone marrow failure, hair loss caused by damaged follicle cells, irregular period or male infertility caused by toxic effect exhibited on ovary or testicle, stomatitis, nausea, dysphagia, and celiac disorder as a side effect caused by disrupted mucous membrane cells of digestive system, diarrhea, nephrotoxicity caused by tubular necrosis, peripheral neuritis or fatigue caused by neurological disorder, vascular disorder like angialgia and rash, and discoloration of skin or finger nails and toe nails. Under the circumstances, it is strongly desired to develop a pharmaceutical agent having increased anticancer effect with minimal adverse side effects.

Phragmitis Rhizoma (noh-geun), which is also referred to as we-geun, is dried roots of a reed (*Phragmitis commnis Trin*) that is a plant belonging to the family *Gramineae.* Reed is found in marsh, river bank, wetland, or sea shore. During spring or autumn, roots of *Phragmitis commnis Trin* are collected, fine roots are removed, washed with water, and dried under the sun. Dried Phragmitis Rhizoma has a flat cylinder shape with glossy surface and yellowish white color. Nodes of the roots are somewhat hard and exhibit distinctive yellowish red color. Vertical wrinkles are present between nodes. Phragmitis Rhizoma has a sweet taste and traditionally it is known to have a cold property and works on lung and stomach meridian. Pharmaceutical effect of Phragmitis Rhizoma includes lowering body heat and stopping nausea by producing phlegm fluid, and it has been used for treating nausea caused by fever, esophagus cancer, or lung abscess, or detoxicating puffer fish toxin. It is also reported to have an activity of blocking ultraviolet rays, causing dark and shiny hair by physiological activity of Phragmitis Rhizoma, there is a report indicating that Phragmitis Rhizoma extract exhibits an influence on glucose, insulin, and lipid synthesis in serum, and it is also reported to have an effect of suppressing the increase of glucagon granules in Langerhans A cells, which is caused by administration of streptozotocin, and suppressing insulin degranulation in Langerhans B cells. It is also reported that methanol extract of Phragmitis Rhizoma has an effect of lowering blood cholesterol level and blood glucose level. It is also reported that methanol extract of Phragmitis Rhizoma root stalk can significantly lower triglyceride concentration in a mouse with hypertriglycemia, hypercholesterolemia, or diabetic hyperlipidemia, and β-sitosterol and p-coumaric acid separated from the methanol extract have an effect of improving serum lipid concentration.

As a technique relating to an extract of Phragmitis Rhizoma, a method for producing an extract with immune-enhancing and anticancer activity from Phragmitis Rhizoma is disclosed in Korean Patent Registration No. 1098875, and a composition for skin moisturization or treatment of dermatitis or atopic dermatitis containing an extract of Phragmitis Rhizoma as an active ingredient is disclosed in Korean Patent Registration No. 1151718. However, a composition containing Phragmitis Rhizoma extract as an active ingredient for prevention, amelioration, or treatment of a disorder caused by side effects of anticancer agents as described in the present invention has never been disclosed.

KR20110126384 discloses an aqueous alcoholic extract of Phragmitis Rhizoma being active as an anticancer agent and as an immunostimulant.

CN103239636 discloses the use of a composition comprising a Phragmitis extract and gingerol with the Phragmitis extract as a main ingredient as an adjuvant to cancer chemotherapy to combat the latter's side effect of vomiting (emesis)

WO 2010/035262 discloses herbal compositions comprising at least four herbal extracts of various types, one type of which may be a Phragmitis extract for use in combating side effects of cancer chemotherapy.

DETAILED DESCRIPTION OF THE INVENTION

TECHNICAL PROBLEMS TO BE SOLVED

The present invention is devised under the circumstances described above. The present invention relates to a composition containing Phragmitis Rhizoma extract as an active ingredient for prevention, amelioration, or treatment of a disorder caused by side effects of anticancer agents, wherein the disorder caused by side effect of an anticancer agent is a hematopoietic toxicity, and wherein the anticancer agent is an antitumor antibiotic, a topoisomerase inhibitor, or a taxane-based anticancer agent and more specifically, by confirming that the survival of hematopoietic stem and progenitor cells is significantly restored as the bone marrow cells of a mouse, which has reduced hematopoietic stem and progenitor cells due to administration of an anticancer agent, are treated with the Phragmitis Rhizoma extract of the present invention, and also bone marrow suppression caused by an anticancer agent is significantly recovered in an animal model having bone marrow suppression that is induced by intraperitoneal injection of an anticancer agent, the present invention is completed.

TECHNICAL MEANS FOR SOLVING THE PROBLEMS

To achieve the purpose described above, the present invention provides a composition containing Phragmitis Rhizoma extract as an active ingredient for use in a method of prevention or treatment of a disorder caused by side effects of anticancer agents, wherein the disorder caused by side effect of an anticancer agent is a hematopoietic toxicity and wherein the anticancer agent is an antitumor antibiotic, a topoisomerase inhibitor or a taxane-based anticancer agent. The present invention further provides a pharmaceutical composition for the above use, which is formulated as a functional health food. The present invention still further provides an anticancer adjuvant agent containing Phragmitis Rhizoma extract as an active ingredient for the above use.

ADVANTAGEOUS EFFECT OF THE INVENTION

The present invention relates to a composition containing Phragmitis Rhizoma extract as an active ingredient for use in a method of prevention or treatment of a disorder caused by side effects of anticancer agents, wherein the disorder caused by side effect of an anticancer agent is a hematopoietic toxicity, and according to the present invention, not only a decrease in hematopoietic stem cell colony caused by an anticancer agent can be significantly restored but also bone marrow suppression caused by an anticancer agent can be significantly recovered so that the Phragmitis Rhizoma extract of the present invention can be used for a pharmaceutical composition, a functional health food, or an anticancer adjuvant agent for prevention or treatment of a disorder caused by side effects of anticancer agents, wherein the disorder caused by side effect of an anticancer agent is a hematopoietic toxicity..

BRIEF DESCRIPTION OF THE DRAWINGS

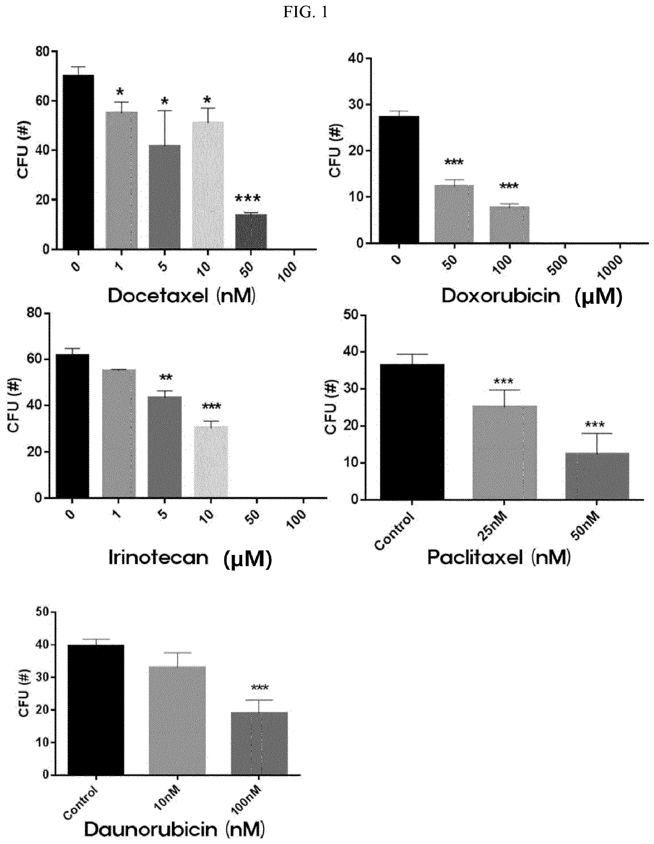

Fig. 1 shows the result of determining the effect of an anticancer agent (docetaxel, doxorubicin, irinotecan, paclitaxel, or daunorubicin) at different concentrations on growth and differentiation of bone marrow cells which have been isolated from a mouse femur, in which the determination was made based on CFU (colony forming unit) assay. In the figure, *, **, and *** indicate that the treatment with an anticancer agent has a statistically significant difference compared to CFU of the control group which has not been treated with any anticancer agent. * means p value of less than 0.05,** means p value of less than 0.01, and *** means p value of less than 0.001.

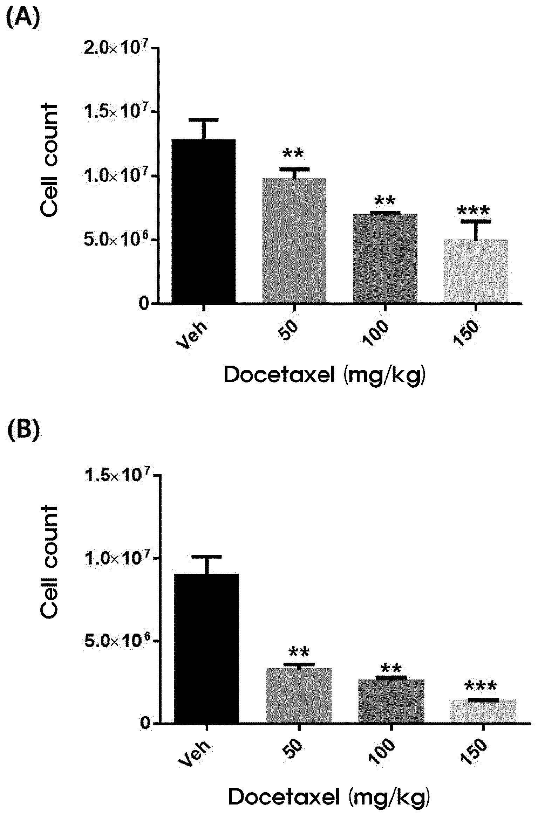

Fig. 2 shows the result of determining the number of bone barrow cells from femur of C5BL/6 mouse after intraperitoneal injection of docetaxel at concentration of 50, 100, or 150 mg/kg, in which the determination was made (A) 3 days or (B) 7 days after the injection. In the figure, ** and *** indicate that the cell survival rate of the docetaxel administration group has a statistically significant difference compared to the control group (Veh) which has not been treated with docetaxel. ** means p value of less than 0.01 and *** means p value of less than 0.001.

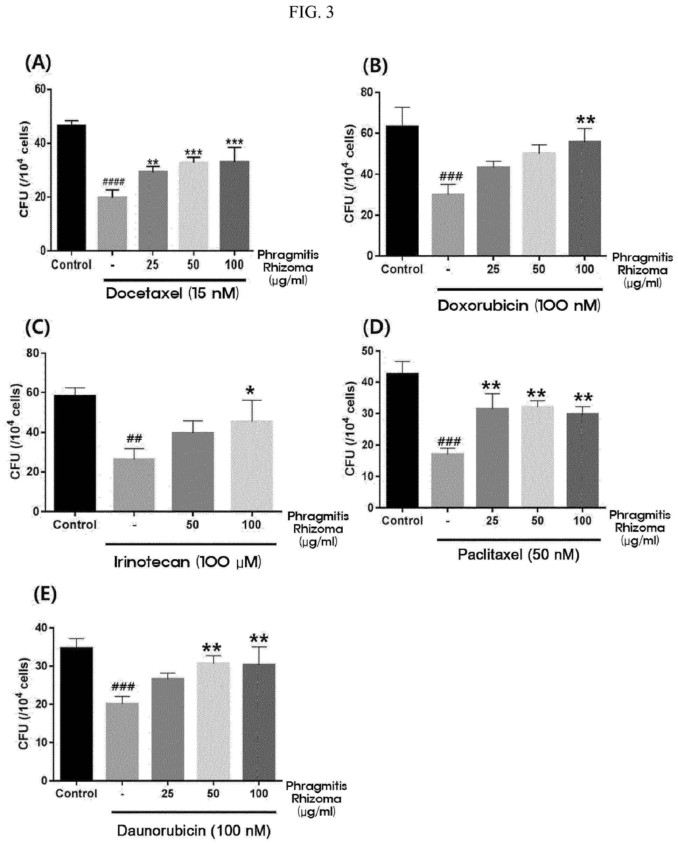

Fig. 3 shows the result of determining a decrease in the number of mouse bone marrow cells caused by a treatment with an anticancer agent (docetaxel, doxorubicin, irinotecan, paclitaxel, or daunorubicin), and the effect of restoring the decrease in the number of mouse bone marrow cells according to a combined treatment of the anticancer agent with the Phragmitis Rhizoma extract of the present invention at concentration of 25, 50, or 100 μg/ml. ##, ###, and #### indicate that the CFU obtained from treatment with an anticancer agent has a statistically significant difference compared to the control group which has not been treated with any anticancer agent, in which ## means p value of less than 0.01, ### means p value of less than 0.001, and #### means p value of less than 0.0001. *, **, and *** indicate that there is a statistically significant difference between the CFU obtained from the group which has been treated with an anticancer agent and the CFU obtained from the group which has been treated with an anticancer agent and the Phragmitis Rhizoma extract in combination, in which * means p value of less than 0.05, ** means p value of less than 0.01, and *** means p value of less than 0.001.

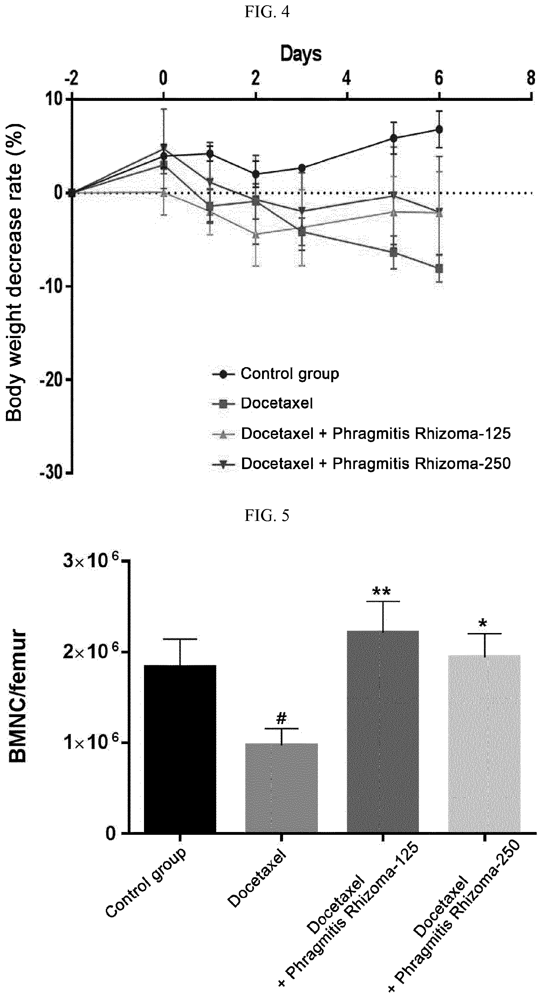

Fig. 4 shows the result of determining a decrease in the body weight of a mouse caused by a treatment with docetaxel, and the effect of suppressing the decrease in the body weight according to a combined treatment of docetaxel with the Phragmitis Rhizoma extract of the present invention. The control group (Control) represents a mouse not treated with any agent, and docetaxel + Phragmitis Rhizoma-125 and docetaxel + Phragmitis Rhizoma-250 mean a mouse received with combined treatment of docetaxel and the Phragmitis Rhizoma extract (125 $\mu$g/ml or 250 $\mu$g/ml, respectively).

Fig. 5 shows the result of determining a decrease in the number of mouse bone marrow cells caused by a treatment with docetaxel, and the effect of suppressing the decrease in the number of mouse bone marrow cells according to a combined treatment of docetaxel (15 nM) with the Phragmitis Rhizoma extract of the present invention at concentration of 125 $\mu$g/ml or 250 $\mu$g/ml. The control group represents a mouse not treated with any agent, and docetaxel + Phragmitis Rhizoma-125 and docetaxel + Phragmitis Rhizoma-250 mean a combined treatment of docetaxel and the Phragmitis Rhizoma extract (125 $\mu$g/ml or 250 $\mu$g/ml). In the figure, # indicates that the number of bone marrow cells after the treatment with docetaxel has a statistically significant difference compared to the control group which has not been treated with docetaxel in which p value is less than 0.05. * and ** indicate that there is a statistically significant difference in the number of bone marrow cells between the docetaxel treatment group and the group which has been treated with docetaxel and the Phragmitis Rhizoma extract in combination, in which * means p value of less than 0.05 and ** means p value of less than 0.01.

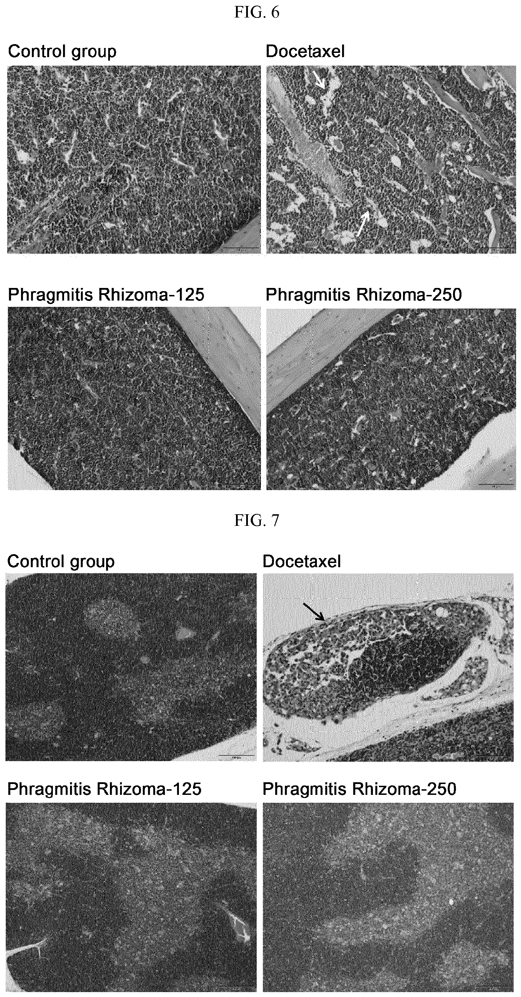

Fig. 6 shows an occurrence of abnormality in bone marrow structure of a mouse that is caused by a treatment with docetaxel (indicated with arrow) while such abnormality hardly occurs after the combined treatment of docetaxel with 125 mg/kg Phragmitis Rhizoma extract (Phragmitis Rhizoma-125) or 250 mg/kg Phragmitis Rhizoma extract (Phragmitis Rhizoma-250), in which the determination was made based on H&E staining.

Fig. 7 shows the result of histopathological analysis of thymus, in which tissue shrinkage and loss of functional lymphoid tissue according to administration of docetaxel are shown (indicated with arrow), and also the recovery from the tissue shrinkage and loss of functional lymphoid organs according to combined treatment of docetaxel with 125 mg/kg Phragmitis Rhizoma extract (Phragmitis Rhizoma-125) or 250 mg/kg Phragmitis Rhizoma extract (Phragmitis Rhizoma-250) is shown.

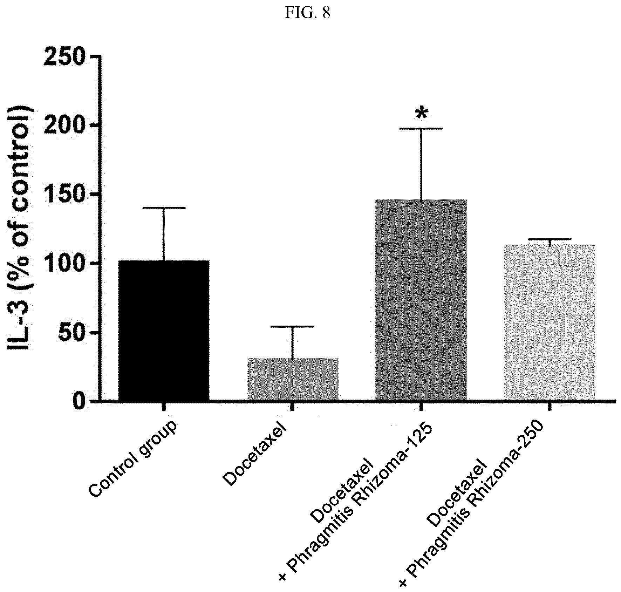

Fig. 8 shows the result of determining the property of Phragmitis Rhizoma extract to alleviate bone marrow suppression in an animal model which has been induced by a treatment with an anticancer agent. Docetaxel + Phragmitis Rhizoma-125 and docetaxel + Phragmitis Rhizoma-250 mean combined administration of docetaxel and Phragmitis Rhizoma 125 mg/kg and docetaxel and Phragmitis Rhizoma 250 mg/kg, respectively. * indicates that there is a statistically significant difference in IL-3 as an immune-stimulating cytokine between the docetaxel treatment group and the group which has been treated with docetaxel and the Phragmitis Rhizoma extract (125 mg/kg) in combination, with p value of less than 0.05.

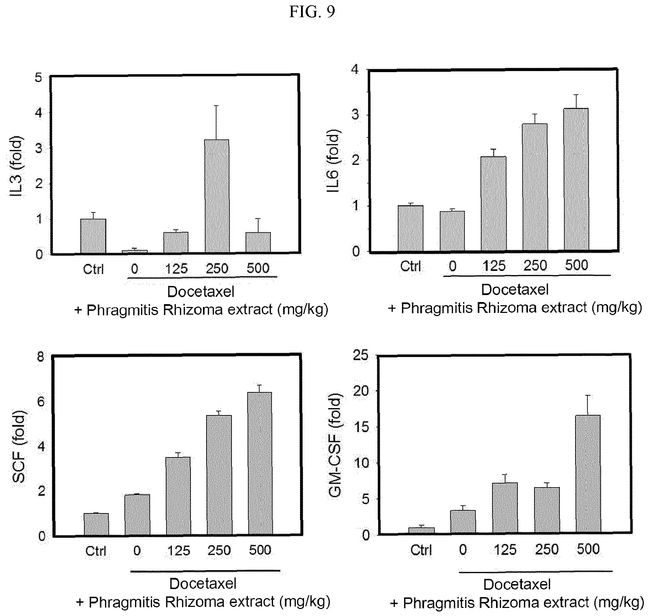

Fig. 9 shows the result of determining a change in expression amount of the immune-stimulating cytokine in mouse spleen cells according to a treatment with the Phragmitis Rhizoma extract.

BEST MODE(S) FOR CARRYING OUT THE INVENTION

The present invention relates to a composition containing Phragmitis Rhizoma extract as an active ingredient for use in a method of prevention or treatment of a disorder caused by side effect of an anticancer agent, wherein the disorder caused by side effect of an anticancer agent is a hematopoietic toxicity, and wherein the anticancer agent is an antitumor antibiotic, a topoisomerase inhibitor, or a taxane-based anticancer agent.

The Phragmitis Rhizoma extract may be produced by a method comprising the following steps, but it is not limited thereto:

1) adding an extracting solvent to Phragmitis Rhizoma followed by extraction,
2) filtering an extract of the step 1), and
3) concentrating a filtered extract of the step 2) under reduced pressure followed by drying to product an extract.

The extracting solvent of the step 1) is preferably water, $C_1$-$C_4$ lower alcohol, or a mixture thereof, but it is not limited thereto.

With regard to the aforementioned production method, any kind of common methods that are generally known as an extraction method in the pertinent art, e.g., filtration, hot water extraction, impregnation extraction, extraction by reflux condensation, and ultrasonic extraction, can be used for obtaining a Phragmitis Rhizoma extract. It is preferable that the extraction is carried out by adding the extracting solvent in an amount of 1 to 20 times the volume of dried Phragmitis Rhizoma, and it is more preferably added in an amount of 3 to 10 times. The extraction temperature is preferably between 20°C and 50°C, but it is not limited thereto. Furthermore, the extraction time is preferably between 10 hours and 100 hours, more preferably between 24 hours and 96 hours, and most preferably 72 hours, but it is not limited thereto. Regarding the above method, the concentration under reduced pressure of the step 3) is preferably carried out by using a vacuum condenser or a vacuum rotary evaporator, but it is not limited thereto. Furthermore, the drying is preferably carried out drying under reduced pressure, drying under vacuum, drying under boiling, spray drying, or freeze drying, but it is not limited thereto.

The cancer is preferably at least one selected from lung cancer, breast cancer, liver cancer, stomach cancer, colon cancer, colorectal cancer, skin cancer, bladder cancer, prostate cancer, ovary cancer, cervical cancer, thyroid cancer, kidney cancer, fibrosarcoma, melanoma, and leukemia, but it is not limited thereto and there is no limitation as long as it is diagnosable cancer.

The anticancer agent is an antitumor antibiotic, a topoisomerase inhibitor, and a taxane-based anticancer agent.

The antitumor antibiotic is preferably at least one selected from a group consisting of actinomycin D, bleomycin sulfate, daunomycin, daunorubicin, doxorubicin, epirubicin, idarubicin, mitomycin, mitomycin-C, and mitramycin, but it is not limited thereto.

The topoisomerase inhibitor is preferably at least one selected from a group consisting of irinotecan, camptothecin, novobiocin, epirubicin, dactinomycin, amsacrine, teniposide, and etoposide, but it is not limited thereto.

The taxane-based anticancer agent is preferably one or both of paclitaxel and docetaxel.

The disorder caused by side effect is hematopoietic toxicity.

The composition for the use of the present invention contains, as an active ingredient, an extract or a fraction of Phragmitis Rhizoma of the present invention in an amount of 0.1 to 99.9% by weight relative to total weight of the composition, and it may also contain a pharmaceutically acceptable carrier, vehicle, or diluent.

The composition for the use of the present invention may be prepared in various formulations including an oral formulation and a parenteral formulation. In case of producing a preparation, production is made by using a diluent or a vehicle such as filler, bulking agent, binding agent, moisturizing agent, disintegrating agent, or surfactant that are commonly used for producing a preparation. As for the solid preparation for oral administration, a tablet, a pill, a powder preparation, a granule, a capsule or the like are included, and such solid preparation is produced by mixing at least one compound with one or more vehicles such as starch, calcium carbonate, sucrose, lactose, or gelatin. Furthermore, other than simple vehicles, a lubricating agent such as magnesium stearate or talc is also used. As for the liquid preparation for oral administration, a suspension, a solution preparation for internal use, an emulsion, a syrup preparation, or the like can be mentioned. Other than water or liquid paraffin as a commonly used simple diluent, various kinds of a vehicle such as moisturizing agent, sweetening agent, aromatic agent, or preservatives may be included. Examples of a preparation for parenteral administration include a sterilized aqueous solution, a non-soluble agent, a suspension agent, an emulsion, a freeze-drying agent, and a suppository agent. As a water insoluble solvent or a suspending agent, propylene glycol, polyethylene glycol, or vegetable oil such as olive oil, and injectable ester such as ethylolate can be used. As a base for a suppository, witepsol, macrogol, tween 61, cacao fat, laurin fat, glycerogelatin, or the like can be used.

The composition for the use of the present invention can be administered either orally or parenterally. In case of parenteral administration, it is preferable to choose external application on skin, intraperitoneal, rectal, intravenous, muscular, subcutaneous, endometrium injection, or intracerebroventricular injection. Most preferably, the composition is used for external application on skin.

The composition for the use of the present invention is administered in a pharmaceutically effective amount. As described herein, the expression "pharmaceutically effective amount" means an amount sufficient for treating a disorder at reasonable benefit-risk ratio that can be applied for a medical treatment. The effective dose level may be determined based on a type or severeness of a disorder, activity of a pharmaceutical, sensitivity to a pharmaceutical, administration period, administration route, excretion ratio, time period for therapy, elements including a pharmaceutical used in combination, and other elements that are well known in the medical field. The composition for the use of the present invention can be administered as a separate therapeutic agent, or it can be used in combination with other therapeutic agent. It can be administered in order or simultaneously with a conventional therapeutic agent. It can be also administered as single-dose or multi-dose. It is important to administer an amount which allows obtainment of the maximum effect with minimum dose while considering the all of the aforementioned elements without having any side effect, and the dosage can be easily determined by a person skilled in the pertinent art.

The dosage of the composition for the use of the present invention may vary depending on body weight, age, sex, health state, diet of a patient, or administration period, administration method, excretion rate, and severity of disorder. However, the daily dosage is, in terms of the amount of the Phragmitis Rhizoma extract, 0.01 to 2,000 mg/kg, preferably 30 to 500 mg/kg, and more preferably 50 to 300 mg/kg, and it can be administered 1 to 6 times per day. The composition for the use of the present invention may be used either singly, or in combination with surgical operation, radiation therapy, hormone therapy, chemotherapy, or therapy using biological response regulator.

The present invention also relates to the above pharmaceutical composition for the above use which is formulated as a functional health food . Extracting solvent for the Phragmitis Rhizoma extract is preferably water, $C_1$-$C_4$ lower alcohol, or a mixture thereof, but it is not limited thereto. The anticancer agent is an antitumor antibiotic, a topoisomerase inhibitor, and a taxane-based anticancer agent. The disorder caused by side effect of an anticancer agent is hematopoietic toxicity.

To the health food for the use of the present invention, the Phragmitis Rhizoma extract may be directly added. Alternatively, it may be used with other food or food ingredients, and suitably used according to a common method. Type of the health food is not particularly limited. Examples of the food to which the Phragmitis Rhizoma extract can be added include meat, sausage, bread, chocolate, candies, snacks, biscuits, pizza, ramen, other noodles, gums, dairy products including ice cream, various kinds of soup, beverage, tea, drink, alcohol beverage, and vitamin complex, and all health foods in general sense are included therein.

The health beverage containing the composition for the use of the present invention may contain, like common beverages, various flavors or natural carbohydrates as an additional component. Examples of the natural carbohydrates include monosaccharides such as glucose or fructose, disaccharides such as maltose or sucrose, polysaccharides such as dextrin or cyclodextrin, and sugar alcohols such as xylitol, sorbitol, or erythritol. As a sweetening agent, a natural sweetening agent such as thaumatin or stevia extract or a synthetic sweetening agent such as saccharine or aspartame can be used. Ratio of the natural carbohydrates is generally about 0.01 to 0.04 g and preferably about 0.02 to 0.03 g relative to 100 g of the composition of the present invention.

Other than those active ingredients that are described above, the health food for the use of the present invention may further contain various kinds of a nutritional agent, vitamins, electrolyte, flavors, a coloring agent, pectinic acid and salts thereof, alginic acid and salts thereof, organic acids, a protective colloid thickening agent, a pH adjusting agent, a stabilizing agent, a preservative, glycerin, alcohol, and a carbonating agent used for carbonate drink. In addition, fruit pulp for producing fruit juice or vegetable juice can be further contained. Those ingredients may be used either independently or in mixture. Ratio of those additives is not particularly critical. However, it is generally selected within a range of from 0.01 to 2 parts by weight relative to 100 parts by weight of the composition of the present invention.

The present invention also relates to anticancer adjuvant agent containing Phragmitis Rhizoma extract as an active ingredient for the above use. The anticancer adjuvant agent is characterized in that it suppresses hematopoietic toxicity, is induced by administration of an anticancer agent.

The anticancer adjuvant agent for the above use may additionally contain one or more active ingredients which exhibit a similar or the same activity as the Phragmitis Rhizoma extract. For clinical administration, the anticancer adjuvant agent can be administered either orally or parenterally. In case of parenteral administration, it can be administered by intraperitoneal injection, intrarectal injection, subcutaneous injection, intravenous injection, intramuscular injection, endometrium injection, intracerebroventricular injection, or intrathoracic injection. It may be also used in the form of a general pharmaceutical preparation.

The anticancer adjuvant agent for the above use may be used either singly, or in combination with surgical operation, radiation therapy, hormone therapy, chemotherapy, or therapy using biological response regulator. The daily dosage of the anticancer adjuvant agent is about 0.0001 to 100 mg/kg, and preferably 0.001 to 10 mg/kg, and it is administered either once or several divided times per day. It may vary depending on body weight, age, sex, health state, diet of a patient, administration period, administration method, excretion rate, and severity of disorder. For actual clinical administration, the anticancer adjuvant agent of the present invention can be administered as various parenteral formulations. In case of producing a preparation, production is made by using a diluent or a vehicle such as filler, bulking agent, binding agent, moisturizing agent, disintegrating agent, or surfactant that are commonly used for producing a preparation. As for the preparation for parenteral administration, sterilized aqueous solution, a non-aqueous preparation, a suspension, an emulsion, a freeze-dried preparation, and a suppository are included. As a non-aqueous solvent or a suspending agent, propylene glycol, polyethylene glycol, or vegetable oil such as olive oil, and injectable ester such as ethylolate can be used. As a base for a suppository, witepsol, macrogol, tween 61, cacao fat, laurin fat, glycerogelatin, or the like can be used.

EXAMPLES

Example 1. Preparation of Phragmitis Rhizoma extract

To prepare a Phragmitis Rhizoma extract as an active ingredient of the present invention, Phragmitis Rhizoma was purchased from Kwangmyungdang Pharmaceuticals (Ulsan, South Korea). One hundred grams of the purchased Phragmitis Rhizoma were crushed, added to a round-bottomed flask, and mixed with 2 liters of water. By heating them in a water bath connected to a reflux extracting device which is equipped with a condenser, extraction was repeatedly carried out 2 times in total, 2 hours for each extraction. The obtained extract was subjected to filtration under reduced pressure by using a paper filter (Whatman No. 2) and a vacuum pump (GAST). By using a rotary evaporator (EYELA), the filtered liquid extract was concentrated under reduced pressure. The concentrated extract was then freeze-dried, and homogenized using a mortar and pestle to obtain Phragmitis Rhizoma extract. The extract was collected and sealed in a plastic container, and then stored in a 4°C low temperature storage till to the test.

Example 2. Determination of hematopoietic toxicity of anticancer agent

In order to determine the hematopoietic toxicity of an anticancer agent currently used for treating cancer patients, the influence of an anticancer agent at various concentrations on growth and differentiation of hematopoietic stem and progenitor cells in mouse bone marrow cells was determined based on an *ex vivo* test and an *in vivo* test.

1) Determination of hematopoietic toxicity of anticancer agent in mouse bone marrow cells (*ex vivo*)

Bone marrow cells were isolated from mouse femur. Subsequently, in a methocult GF M3434 medium containing recombinant murine stem cell factor (rm stem cell factor), rm IL-3, recombinant human IL-6 (rh IL-6), and rh erythropoietin to which docetaxel, doxorubicin, irinotecan, paclitaxel, or daunorubicin is added at various concentrations, the above mouse bone marrow cells were cultured for 7 to 10 days to induce the growth of hematopoietic stem and progenitor cells. The grown hematopoietic stem and progenitor cells were collected, and by carrying out CFU assay (i.e., Colony Forming Unit assay), the influence exhibited by an anticancer agent on growth and differentiation of the hematopoietic stem and progenitor cells was examined.
As a result, the hematopoietic toxicity was shown even at low concentrations as shown in Fig. 1. In particular, it was found that very significant hematopoietic toxicity is exhibited with docetaxel of 50 nM or higher, doxorubicin of 50 $\mu$M or higher, irinotecan of 5 $\mu$M or higher, paclitaxel of 25 nM or higher or daunorubicin of 100 nM or higher.

2) Determination of hematopoietic toxicity in mouse having intraperitoneal injection of anticancer agent (*in vivo*)

A C57BL/6 mouse was subjected to intraperitoneal injection of docetaxel at 50, 100, or 150 mg/kg. Three days and seven days after the injection, bone marrow cells were isolated from the mouse femur and counted, and the survival rate was measured.
As a result, from any case of having docetaxel injection at 50, 100, or 150 mg/kg, a decrease in the number of the isolated bone marrow cells was observed in significant sense, three days and seven days after the injection, as shown in Fig. 2. Accordingly, it was confirmed that the hematopoietic toxicity is exhibited in an *in vivo* animal model.

Example 3. Determination of effect of recovering bone marrow toxicity by Phragmitis Rhizoma extract against bone marrow toxicity caused by anticancer agent

In order to determine whether or not the Phragmitis Rhizoma extract has an effect of ameliorating the hematopoietic toxicity that has been induced by an anticancer agent (i.e., 15 nM docetaxel, 100 nM doxorubicin, 100 $\mu$M irinotecan, 50 nM paclitaxel, or 100 nM daunorubicin) in bone marrow cells, a combined treatment of the Phragmitis Rhizoma extract with 15 nM docetaxel, 100 nM doxorubicin, 100 $\mu$M irinotecan, 50 nM paclitaxel, or 100 nM daunorubicin was carried out and the effect of ameliorating the hematopoietic toxicity was examined.
Specifically, bone marrow cells were separated from mouse femur, and then treated, in a methocult GF M3434 medium containing rm stem cell factor, rm IL-3, recombinant human IL-6, and rh erythropoietin, with an anticancer agent like 15 nM docetaxel as an agent inducing the hematopoietic toxicity in bone marrow cells. After that, the Phragmitis Rhizoma extract with concentration of 25, 50, or 100 $\mu$g/ml prepared in above Example 1 was added thereto, and growth of the hematopoietic stem and progenitor cells was induced according to culture for 7 days. After 7 days, the grown hematopoietic stem and progenitor cells were collected, and subjected to CFU assay to determine the growth of the hematopoietic stem and progenitor cells. As a negative control, the cells were treated with a solvent (i.e., PBS containing 0.5% DMSO) instead of the Phragmitis Rhizoma extract, and the same treatment as above was carried out.
As a result, as shown in Fig. 3, it was confirmed that the decrease in colony of hematopoietic stem and progenitor cells, which has been induced by each anticancer agent, is recovered in a statistically significant sense by the Phragmitis Rhizoma extract (Fig. 3).

Example 4. Determination of alleviation of bone marrow suppression by Phragmitis Rhizoma extract in animal model having bone marrow suppression induced by docetaxel

In order to determine whether or not the Phragmitis Rhizoma extract exhibits an effect of ameliorating the hematopoietic toxicity in an animal model having bone marrow suppression, intraperitoneal injection of docetaxel to a mouse was carried out to induce bone marrow suppression. After that, a change brought by the Phragmitis Rhizoma extract was determined.

Specifically, C57BL/6 mice were categorized into 4 groups. Group 1 is a control, i.e., a group treated with saline containing 5% ethanol and 2% polysorbate 80. Group 2 is a docetaxel treatment group, i.e., group with intraperitoneal injection (3 times) of 30 mg/kg docetaxel. Group 3 is a docetaxel and 125 mg/kg Phragmitis Rhizoma extract administration group, i.e., a group to which compulsory administration of 125 mg/kg Phragmitis Rhizoma extract was carried out, once a day starting from 2 days before the administration of docetaxel, and the compulsory administration of 125 mg/kg Phragmitis Rhizoma extract (once a day) was continued even for the 3 days of administering docetaxel. Lastly, Group 4 is a docetaxel and 250 mg/kg Phragmitis Rhizoma extract administration group, i.e., a group to which administration of 250 mg/kg Phragmitis Rhizoma extract was carried out, in the same manner as above Group 3, once a day starting from 2 days before the administration of docetaxel, and the compulsory administration of 250 mg/kg Phragmitis Rhizoma extract (once a day) was continued even for the 3 days of administering docetaxel.

After that, a change in body weight of the mouse belonging to each of the control group, docetaxel administration group, combined administration group with docetaxel and 125 mg/kg Phragmitis Rhizoma extract, and combined administration group with docetaxel and 250 mg/kg Phragmitis Rhizoma extract was measured every day. On Day 6, the mouse was sacrificed and the weight of the spleen and thymus, which play an important role in determining cell number change in bone marrow and hematopoiesis, was measured.

As a result, when the mouse was administered with docetaxel, a significant decrease in body weight was induced along with hematopoietic toxicity and bone marrow suppression as shown in Fig. 4 and Fig. 5. However, according to the combined administration of Phragmitis Rhizoma extract, the body weight decrease was alleviated (Fig. 4). Furthermore, while the number of bone marrow cells (i.e., bone marrow mononuclear cells) was decreased significantly in the docetaxel administration group compared to the control, recovery from the bone marrow suppression was observed from the group administered with the Phragmitis Rhizoma extract (Fig. 5).

As a result of carrying out a histological analysis of a femur of mouse belonging to each administration group described above, it was found that the group administered with docetaxel only showed a hypo cellularity and an abnormality in cellular structure in bone marrow (indicated with yellow arrow) as caused by a decrease in hematopoietic cells. However, in the combined administration group with Phragmitis Rhizoma extract, those phenomena were found to be rather diminished (Fig. 6). Although not directly related to the invention as claimed, from the result of the histopathological analysis of the mouse thymus, it was also found that the organ shrinkage and loss of functional lymphoid organ, which have been caused by docetaxel administration, are recovered according to the combined administration of Phragmitis Rhizoma extract (Fig. 7).

Furthermore, in case of the mouse administered with docetaxel, an abnormality in the spleen and thymus as an organ involved with hematopoiesis was caused, thus showing a decrease in organ index as a result of a dramatic reduction in weight. However, it was found that the decrease in organ index is reversed in the group to which docetaxel and the Phragmitis Rhizoma extract are administered in combination.

$$\text{Organ index (mg/g)} = \text{Weight of organ (mg)}/\text{Weight of animal (g)}$$

From the group administered with docetaxel, shrinkage of spleen and thymus as an organ involved with the hematopoiesis, and weight decrease were observed. However, from the group administered with the Phragmitis Rhizoma extract, the effect of reversing such decrease was confirmed (Table 1).

[Table 1]

| Organ index for determining change in weight of spleen and thymus of animal belonging to docetaxel administration group or combined administration group withdocetaxel and Phragmitis Rhizoma extract | | |
|---|---|---|
| | Spleen index (mg/g) | Thymus index (mg/g) |
| Control group | 3.111±0.58 | 2.360±0.28 |
| Docetaxel | 2.366±0.22 | 0.911±0.22 |
| Docetaxel + Phragmitis Rhizoma-125 | 3.004±0.25 | 1.721±0.07 |
| Docetaxel + Phragmitis Rhizoma-250 | 2.842±0.27 | 1.842±0.49 |

Meanwhile, from a mouse spleen tissue, mRNA was separated and a change in expression of cytokine, which is involved with the hematopoiesis, was determined. According to the administration of docetaxel, the hematopoiesis, in particular, expression of IL-3 deeply involved with differentiation and proliferation of myeloid progenitor cells, has largely decreased. On the contrary, according to combined administration of docetaxel and Phragmitis Rhizoma extract, the decreased IL-3 expression is recovered (Fig. 8).

Example 5. Determination (*in vivo*) of influence of Phragmitis Rhizoma extract on immune-stimulating cytokine in mouse spleen cells

In order to determine the influence of Phragmitis Rhizoma extract on expression of cytokine involved with hematopoiesis in an animal mold having bone marrow suppression, bone marrow suppression was induced by intraperitoneal injection of docetaxel to a mouse. After that, a change brought by the Phragmitis Rhizoma extract was determined.

Specifically, C57BL/6 mice were categorized into 5 groups. Group 1 is a control, i.e., a group treated with saline containing 5% ethanol and 2% polysorbate 80. Group 2 is a docetaxel treatment group, i.e., a group with intraperitoneal injection (3 times) of 30 mg/kg docetaxel. Group 3 is a docetaxel and 125 mg/kg Phragmitis Rhizoma extract administration group, i.e., a group to which compulsory administration of 125 mg/kg Phragmitis Rhizoma extract was carried out, once a day starting from 2 days before the administration of docetaxel, and the compulsory administration of 125 mg/kg Phragmitis Rhizoma extract (once a day) was continued even for the 3 days of administering docetaxel. Group 4 is a docetaxel and 250 mg/kg Phragmitis Rhizoma extract administration group, i.e., a group to which administration was carried out in the same manner as above Group 3. Lastly, Group 5 is a docetaxel and 500 mg/kg Phragmitis Rhizoma extract administration group, i.e., a group to which administration was carried out in the same manner as Group 3.

Spleen tissues were collected from the animal and mRNA was extracted therefrom. Then, as a result of determining a change in expression of cytokine involved with hematopoiesis, the increased expression of IL-3, IL-6, SCF (stem cell factor), and GM-CSF (granulocyte-macrophage colony-stimulating factor), which are the cytokines promoting the differentiation and proliferation of hematopoietic cells, was confirmed (Fig. 9).

Example 6. Determination of influence of Phragmitis Rhizoma extract on animal model having docetaxel-induced bone marrow suppression

In order to determine the influence of Phragmitis Rhizoma extract on blood parameters of an animal model having bone marrow suppression, bone marrow suppression was induced by intraperitoneal injection of docetaxel to a mouse. After that, a change brought by the Phragmitis Rhizoma extract was determined.

Specifically, C57BL/6 mice were categorized into 5 groups. Group 1 is a control, i.e., a group treated with saline containing 5% ethanol and 2% polysorbate 80. Group 2 is a docetaxel treatment group, i.e., a group with intraperitoneal injection (3 times) of 30 mg/kg docetaxel. Group 3 is a docetaxel and 30 mg/kg Phragmitis Rhizoma extract administration group, i.e., a group to which compulsory administration of 30 mg/kg Phragmitis Rhizoma extract was carried out, once a day starting from 2 days before the administration of docetaxel, and the compulsory administration of 30 mg/kg Phragmitis Rhizoma extract (once a day) was continued even for the 3 days of administering docetaxel. Group 4 is a docetaxel and 100 mg/kg Phragmitis Rhizoma extract administration group, i.e., a group to which administration was carried out in the same manner as above Group 3. Lastly, Group 5 is a docetaxel and 300 mg/kg Phragmitis Rhizoma extract administration group, i.e., a group to which administration was carried out in the same manner as Group 3.

Blood was taken from the animal and a change in the number of blood cells was determined. As a result, a decrease in the number of the white blood cells, neutrophils, lymphocytes, and red blood cells was shown from the docetaxel administration group. However, a recovery from such decrease was obtained from the group administered with the Phragmitis Rhizoma extract in combination.

[Table 2]

|  | WBCs | Neutrophils | Lymphocytes | RBCs |
|---|---|---|---|---|
| Control group | 3.44±0.24 | 0.48±0.02 | 2.88±0.23 | 9.11±0.18 |
| Docetaxel Group | 2.09±0.19 | 0.30±0.01 | 1.56±0.22 | 7.72±0.15 |
| Docetaxel + Phragmitis Rhizoma (30 mg/kg) | 2.52±0.15 | 0.47±0.06## | 1.83±0.10 | 8.04±0.13 |
| Docetaxel + Phragmitis Rhizoma (100 mg/kg) | 2.59±0.13# | 0.41±0.06 | 2.09±0.10# | 7.88±0.13 |
| Docetaxel + Phragmitis Rhizoma (300 mg/kg) | 2.84±0.30 | 0.42±0.07 | 2.22±0.25 | 8.11±0.18 |

The data of above Table 2 represent mean ± standard error (SEM) of the value obtained from ten mice, and # and ## mean $p < 0.05$ and $p < 0.01$, respectively, vs. docetaxel administration group.

Claims

1. A pharmaceutical composition containing Phragmitis Rhizoma extract as an active ingredient for use in a method of prevention or treatment of a disorder caused by side effect of an anticancer agent, wherein the disorder caused by side effect of an anticancer agent is a hematopoietic toxicity, and wherein the anticancer agent is an antitumor antibiotic, a topoisomerase inhibitor, or a taxane-based anticancer agent.

2. The pharmaceutical composition for the use according to Claim 1, wherein the extract has been extracted by using water, $C_1$-$C_4$ lower alcohol, or a mixture thereof as a solvent.

3. The pharmaceutical composition for the use according to Claim 1, wherein the antitumor antibiotic is at least one selected from a group consisting of actinomycin D, bleomycin sulfate, daunomycin, daunorubicin, doxorubicin, epirubicin, idarubicin, mitomycin, mitomycin-C, and mitramycin.

4. The pharmaceutical composition for the use according to Claim 1, wherein the topoisomerase inhibitor is at least one selected from a group consisting of irinotecan, camptothecin, novobiocin, epirubicin, dactinomycin, amsacrine, teniposide, and etoposide.

5. The pharmaceutical composition for the use according to Claim 1, wherein the taxane-based anticancer agent is at least one selected from paclitaxel and docetaxel.

6. The pharmaceutical composition for the use according to claim 1, which is formulated as a functional health food.

7. An anticancer adjuvant agent containing Phragmitis Rhizoma extract as an active ingredient for use in a method of prevention or treatment of a hematopoietic toxicity, wherein the Phragmitis Rhizoma extract ameliorates the hematopoietic toxicity that is induced by administration of an anticancer agent, wherein the anticancer agent is an antitumor antibiotic, a topoisomerase inhibitor or a taxane based anticancer agent.

**Patentansprüche**

1. Pharmazeutische Zusammensetzung, enthaltend Phragmitis Rhizoma-Extrakt als Wirkstoff zur Verwendung in einem Verfahren zur Vorbeugung oder Behandlung einer durch eine Nebenwirkung eines Antikrebsmittels verursachten Störung, wobei die durch eine Nebenwirkung eines Antikrebsmittels verursachte Störung eine hämatopoetische Toxizität ist und wobei das Antikrebsmittel ein Antitumor-Antibiotikum, ein Topoisomerase-Inhibitor oder ein Antikrebsmittel auf Taxanbasis ist.

2. Pharmazeutische Zusammensetzung für die Verwendung nach Anspruch 1, wobei der Extrakt unter Verwendung von Wasser, $C_1$-$C_4$-Niederalkohol oder einer Mischung davon als Lösungsmittel extrahiert wurde.

3. Pharmazeutische Zusammensetzung für die Verwendung nach Anspruch 1, wobei das Antitumor-Antibiotikum mindestens eines ist, das aus einer Gruppe ausgewählt ist, die aus Actinomycin D, Bleomycinsulfat, Daunomycin, Daunorubicin, Doxorubicin, Epirubicin, Idarubicin, Mitomycin, Mitomycin-C und Mitramycin besteht.

4. Pharmazeutische Zusammensetzung für die Verwendung nach Anspruch 1, wobei der Topoisomerase-Inhibitor mindestens einer ist, ausgewählt aus einer Gruppe bestehend aus Irinotecan, Camptothecin, Novobiocin, Epirubicin, Dactinomycin, Amsacrin, Teniposid und Etoposid.

5. Pharmazeutische Zusammensetzung für die Verwendung nach Anspruch 1, wobei das auf Taxan basierende Antikrebsmittel mindestens eines ist, ausgewählt aus Paclitaxel und Docetaxel.

6. Pharmazeutische Zusammensetzung für die Verwendung nach Anspruch 1, die als ein funktionelles Gesundheitsnahrungsmittel formuliert ist.

7. Antikrebshilfsmittel, enthaltend Phragmitis Rhizoma-Extrakt als Wirkstoff zur Verwendung in einem Verfahren zur Vorbeugung oder Behandlung einer hämatopoetischen Toxizität, wobei der Phragmitis Rhizoma-Extrakt die hämatopoetische Toxizität, die durch die Verabreichung eines Antikrebsmittels induziert wird, verbessert, wobei das Antikrebsmittel ein Antitumor-Antibiotikum, ein Topoisomerase-Inhibitor oder ein Antikrebsmittel auf Taxanbasis ist.

# EP 3 417 869 B1

**Revendications**

1. Composition pharmaceutique contenant de l'extrait de Phragmitis Rhizoma en tant qu'ingrédient actif à utiliser dans une méthode de prévention ou de traitement d'un trouble causé par l'effet secondaire d'un agent anticancéreux, dans laquelle le trouble causé par l'effet secondaire d'un agent anticancéreux est une toxicité hématopoïétique, et dans laquelle l'agent anticancéreux est un antibiotique antitumoral, un inhibiteur de topoisomérase, ou un agent anticancéreux à base de taxane.

2. Composition pharmaceutique pour l'utilisation selon la revendication 1, dans laquelle l'extrait a été extrait en utilisant de l'eau, un alcool inférieur en $C_1$-$C_4$ ou un mélange de ceux-ci comme solvant.

3. Composition pharmaceutique pour l'utilisation selon la revendication 1, dans laquelle l'antibiotique antitumoral est au moins un antibiotique choisi dans le groupe constitué de l'actinomycine D, du sulfate de bléomycine, de la daunomycine, de la daunorubicine, de la doxorubicine, de l'épirubicine, de l'idarubicine, de la mitomycine, de la mitomycine-C et de la mitramycine.

4. Composition pharmaceutique pour l'utilisation selon la revendication 1, dans laquelle l'inhibiteur de topoisomérase est au moins un inhibiteur choisi dans le groupe constitué par l'irinotécan, la camptothécine, la novobiocine, l'épirubicine, la dactinomycine, l'amsacrine, le téniposide et l'étoposide.

5. Composition pharmaceutique pour l'utilisation selon la revendication 1, dans laquelle l'agent anticancéreux à base de taxane est au moins un agent choisi parmi le paclitaxel et le docétaxel.

6. Composition pharmaceutique pour l'utilisation selon la revendication 1, qui est formulée comme un aliment de santé fonctionnel.

7. Agent adjuvant anticancéreux contenant de l'extrait de Phragmitis Rhizoma comme ingrédient actif pour utilisation dans une méthode de prévention ou de traitement d'une toxicité hématopoïétique, dans laquelle l'extrait de Phragmitis Rhizoma améliore la toxicité hématopoïétique induite par l'administration d'un agent anticancéreux, dans lequel l'agent anticancéreux est un antibiotique antitumoral, un inhibiteur de topoisomérase ou un agent anticancéreux à base de taxane.

FIG. 1

**(A)**

**(B)**

FIG. 3

FIG. 4

FIG. 5

FIG. 6

Control group

Docetaxel

Phragmitis Rhizoma-125

Phragmitis Rhizoma-250

FIG. 7

Control group

Docetaxel

Phragmitis Rhizoma-125

Phragmitis Rhizoma-250

FIG. 8

FIG. 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1098875
- KR 1151718
- KR 20110126384
- CN 103239636
- WO 2010035262 A